# EUROPÄISCHE PATENTANMELDUNG

(11) **EP 1 792 593 A1**
(43) Veröffentlichungstag der Anmeldung: **06.06.2007**
(21) Anmeldenummer: 05026260.9
(22) Anmeldetag: 01.12.2005
(51) Int. Cl.: A61F 9/01

(54) **Lasereinrichtung für die refraktive Augenbehandlung**

(71) Anmelder: WaveLight Laser Technologie AG, 91058 Erlangen (DE)
(72) Erfinder: Donitzky, Christof, 90542 Eckental (DE); Vogler, Klaus, Dr., 90542 Eckental (DE)
(74) Vertreter: von Hellfeld, Axel

(57) **Zusammenfassung**

Eine Lasereinrichtung (10) für die rekfraktive Augenbehandlung umfasst eine erste Laserstrahlungsquelle (14), deren bereitgestellte Laserstrahlung auf die Anbringung eines Hornhautschnitts abgestimmt ist, eine zweite Laserstrahlungsquelle (16), deren bereitgestellte Laserstrahlung auf die Ablation von Hornhautmaterial abgestimmt ist, sowie Optikmittel (28-46) zur Führung und Formung der Laserstrahlung beider Laserstrahlungsquellen. Erfindungsgemäß definieren die Optikmittel einen der Laserstrahlung beider Laserstrahlungsquellen (14, 16) gemeinsam zugeordneten optischen Pfadabschnitt (48), über welchen die Laserstrahlung jeder Laserstrahlungsquelle auf ein zu behandelndes Auge (52) trifft.

## Beschreibung

Die Erfindung betrifft eine Lasereinrichtung für die refraktive Augenbehandlung.

Für die Korrektur von Fehlsichtigkeiten des menschlichen Auges, etwa von Myopie, Hyperopie und Astigmatismus, ist es bekannt, vom Epithel der Hornhaut ein in der Fachwelt als Flap bezeichnetes Scheibchen abzulösen und anschließend das darunterliegende Hornhautgewebe mittels Laserstrahlung gemäß einem auf die zu behebende Fehlsichtigkeit abgestimmten Ablationsprofil zu ablatieren (abzutragen). Der Flap wird dabei so präpariert, dass er an einer Stelle seines Rands noch mit der Hornhaut verbunden ist. Für die Freilegung des zu ablatierenden Hornhautgewebes genügt es dann, den Flap zur Seite zu klappen. Nach erfolgter Photoablation des Hornhautgewebes wird der Flap wieder zurückgeklappt, was eine rasche Wundheilung ermöglicht.

Zur Präparation des Flaps sind verschiedene Techniken bekannt. Eine dieser Techniken beinhaltet die Anbringung eines Hornhautschnitts mittels gepulster Laserstrahlung, deren Pulslänge im Bereich von Femtosekunden liegt. Gängigerweise wird dabei Laserstrahlung mit einer Wellenlänge im kurzwelligen Infrarot-Bereich verwendet, beispielsweise zwischen 1000 und 1080 nm. Die Strahlung wird auf einen Ort im Innern des Hornhautgewebes fokussiert, beispielsweise in einer Tiefe zwischen 90 und 160 µm unterhalb der Hornhautoberfläche, so dass ein entsprechend dicker Flap erzeugt werden kann. Zur Anbringung des Hornhautschnitts wird die solchermaßen fokussierte Laserstrahlung mittels einer geeignet gesteuerten Ablenkeinrichtung (Scanner) entlang des Hornhautbereichs geführt, in dem die Gewebsablösung bewirkt werden soll. Aneinadergereihte winzige Photodisruptionen bewirken im Fokus der Laserstrahlung (ein üblicher Fokusdurchmesser ist annähernd 10 µm) eine Durchtrennung des Gewebes und in der Gesamtheit einen stromalen Schnitt.

Für die Photoablation von Hornhautgewebe mit dem Ziel der Neuformung der Kornea eignet sich dagegen besonders UV-Licht, für dessen Erzeugung häufig Excimerlaser zum Einsatz kommen. In der Regel wird ein bei einer Wellenlänge von 193 nm emittierender ArF-Excimerlaser verwendet. Diese Wellenlänge hat sich bewährt und ist medizinisch erprobt. Auch bei der Ablation ist die verwendete Laserstrahlung üblicherweise gepulst. Die Pulsdauern sind jedoch typischerweise wesentlich länger als bei der für den Hornhautschnitt verwendeten Laserstrahlung; sie liegen gemeinhin in der Größenordnung von Nanosekunden.

Für eine Augenbehandlung der vorstehenden Art werden demnach zwei Arten von Laserstrahlung benötigt, die sich zumindest durch ihre Wellenlänge voneinander unterscheiden. Herkömmlicherweise sind die Operationspraxen, in denen solche Augenbehandlungen durchgeführt werden, mit zwei vollständig getrennten Lasersystemen ausgestattet, deren eines eine Laserstrahlungsquelle besitzt, die für die Anbringung des Hornhautschnitts geeignete Laserstrahlung erzeugt, und deren anderes eine weitere Laserstrahlungsquelle besitzt, die für die Gewebsablation geeignete Laserstrahlung bereitstellt. Jedes der Lasersysteme ist dabei mit allen Komponenten und Funktionen ausgestattet, um autark zu arbeiten, also unabhängig vom jeweils anderen Lasersystem. Das heißt, jedes Lasersystem enthält seine eigene Betriebsenergieversorgung, seine eigene Steuerung, seine eigenen Optikmittel für die Strahlformung und -führung sowie gegebenenfalls seine eigene Kühlung. Es ist leicht vorstellbar, dass solche Lasersysteme Apparaturen beträchtlicher Größe sind. Aufgrund ihrer Ausmaße und ihres Gewichts sind sie in den Operationspraxen stationär aufgestellt, und zwar räumlich getrennt voneinander, weswegen der Patient zwischen den beiden Operationsschritten der Anbringung des Hornhautschnitts und der anschließenden Ablation des Hornhautgewebes umgelagert werden muss. Dies ist ungünstig, da die beiden Operationsschritte möglichst in einem kontinuierlichen, ununterbrochenen Vorgang durchgeführt werden sollten, und zwar in möglichst kurzer Zeit. Die Umlagerung des Patienten mitten in dem chirurgischen Eingriff ist für den Patienten äußerst unangenehm und stellt ein zusätzliches Risiko dar, denn zu diesem Zeitpunkt kann er mit dem zu behandelnden Auge nicht mehr sehen. Auch für den Operateur ist die Umlagerung des Patienten eine unerwünschte Unterbrechung im Operationsfluss, die womöglich seine Konzentration beeinträchtigen und damit das Operationsergebnis gefährden kann.

Aufgabe der Erfindung ist es daher, eine Lasereinrichtung für die refraktive Augenbehandlung bereitzustellen, die einen vereinfachten Operationsablauf und infolgedessen eine erhöhte Gewähr für gute Behandlungsergebnisse bieten kann.

Bei der Lösung dieser Aufgabe geht die Erfindung aus von einer Lasereinrichtung für die refraktive Augenbehandlung, umfassend eine erste Laserstrahlungsquelle, deren bereitgestellte Laserstrahlung auf die Anbringung eines Hornhautschnitts abgestimmt ist, eine zweite Laserstrahlungsquelle, deren bereitgestellte Laserstrahlung auf die Ablation von Hornhautmaterial abgestimmt ist, sowie Optikmittel zur Führung und Formung der Laserstrahlung beider Laserstrahlungsquellen.

Erfindungsgemäß ist dabei vorgesehen, dass die Optikmittel einen der Laserstrahlung beider Laserstrahlungsquellen gemeinsam zugeordneten optischen Pfadabschnitt definieren, über welchen die Laserstrahlung jeder Laserstrahlungsquelle auf ein zu behandelndes Auge trifft. Vorteilhafterweise wird dabei angestrebt, dass beide Arten der Laserstrahlung ohne Nachjustage auf das gleiche Behandlungsgebiet treffen. Dies kann beispielsweise dadurch geschehen, dass beide Strahlungen kolinear durch identische Optiken gehen oder in getrennten Strahlführungen (mit verschiedenen Optiken) auf den gleichen Behandlungsort geführt werden.

Die Erfindung stellt eine Lasereinrichtung bereit, welche die optischen Pfade der Laserstrahlung beider Laserstrahlungsquellen zusammenführt, so dass die Laserstrahlung jeder Laserstrahlungsquelle durch ein gemeinsames Austrittsfenster aus der Lasereinrichtung austreten und auf das zu behandelnde Auge gelangen kann. Auf diese Weise ist keine Umlagerung des Patienten nach der Anbringung des Hornhautschnitts mehr nötig; er kann auch für die nachfolgende Ablation so gebettet und positioniert bleiben, wie er es für die Anbringung des Hornhautschnitts war. Der gesamte Operationsvorgang wird hierdurch vereinfacht und verkürzt, was der Behandlungsqualität zu Gute kommt und das Risiko, welches eine Unterbrechung der Operation beinhaltet, vermeidet.

Die für die Lasereinrichtung benötigte Aufstellfläche kann insgesamt reduziert werden, wenn die beiden Laserstrahlungsquellen und gewünschtenfalls auch die Optikmittel in gemeinsamen Gehäusemitteln untergebracht sind. Dieser Aspekt wird auch unabhängig von der Zusammenführung der optischen Pfade der von den beiden Laserstrahlungsquellen emittierten Laserstrahlung als schutzfähig angesehen und dient ebenfalls dem Ziel, den Patienten nach der Anbringung des Hornhautschnitts nicht mehr durch den Operationsraum transportieren zu müssen, um anschließend die Ablation des Hornhautgewebes vornehmen zu können. Denn die Zusammenfassung der Laserstrahlungsquellen und gewünschtenfalls der Optikmittel in einem gemeinsamen Gehäuse ermöglicht ein kompaktes, integriertes Lasersystem, mit dem beide Arten der Laserbehandlung, also die Anbringung des Hornhautschnitts und die Gewebsablation, an einem Ort durchgeführt werden können. Dabei ist es möglich, das Lasersystem mit einem einzigen Austrittsfenster auszubilden, durch das die Laserstrahlung beider Laserstrahlungsquellen austritt und auf das Patientenauge trifft. Grundsätzlich vorstellbar ist jedoch auch eine Lösung, bei der gesonderte, zweckmäßigerweise vergleichsweise nah beieinanderliegende Austrittsfenster vorgesehen sind, durch welche die Laserstrahlung jeweils einer der Laserstrahlungsquellen auf das Patientenauge geleitet wird.

Die Erfindung ermöglicht Synergien, da zumindest ein Teil der für den Betrieb und die Steuerung der Laserstrahlungsquellen und die Führung und Fokussierung der emittierten Laserstrahlung benötigten Komponenten beiden Laserstrahlungsquellen gemeinsam zugeordnet sein können. So ist es beispielsweise denkbar, eine gemeinsame Betriebsenergieversorgungseinheit oder/und ein gemeinsames Kühlagregat oder/und eine gemeinsame Steuereinheit für beide Laserstrahlungsquellen vorzusehen. Alternativ oder zusätzlich ist es vorstellbar, eine gemeinsame Ablenkeinheit (Scanner) oder/und eine gemeinsame Fokussiereinheit für die Laserstrahlung beider Laserstrahlungsquellen vorzusehen. In diesem Fall kann die Zusammenführung der optischen Pfade schon vor dem gemeinsamen Scanner beziehungsweise vor der gemeinsamen Fokussiereinheit stattfinden.

Es ist freilich auch möglich, dass die Optikmittel in Zuordnung zu jeder der Laserstrahlungsquellen je eine Strahlfokussiereinheit umfassen, wobei der gemeinsame optische Pfadabschnitt dann im Strahlengang nach den beiden Strahlfokussiereinheiten beginnt. Die Zusammenführung der optischen Pfade der Laserstrahlung beider Laserstrahlungsquellen erfolgt also bei dieser Ausführungsform erst nach der Strahlfokussierung.

Die Erfindung wird nachstehend anhand der beigefügten Zeichnungen weiter erläutert. Es stellen dar:
Figur 1 in schematischer Blockdarstellung ein erstes Ausführungsbeispiel einer erfindungsgemäßen Lasereinrichtung und
Figur 2 in gleichfalls schematischer Blockdarstellung ein weiteres Ausführungsbeispiel einer erfindungsgemäßen Lasereinrichtung.

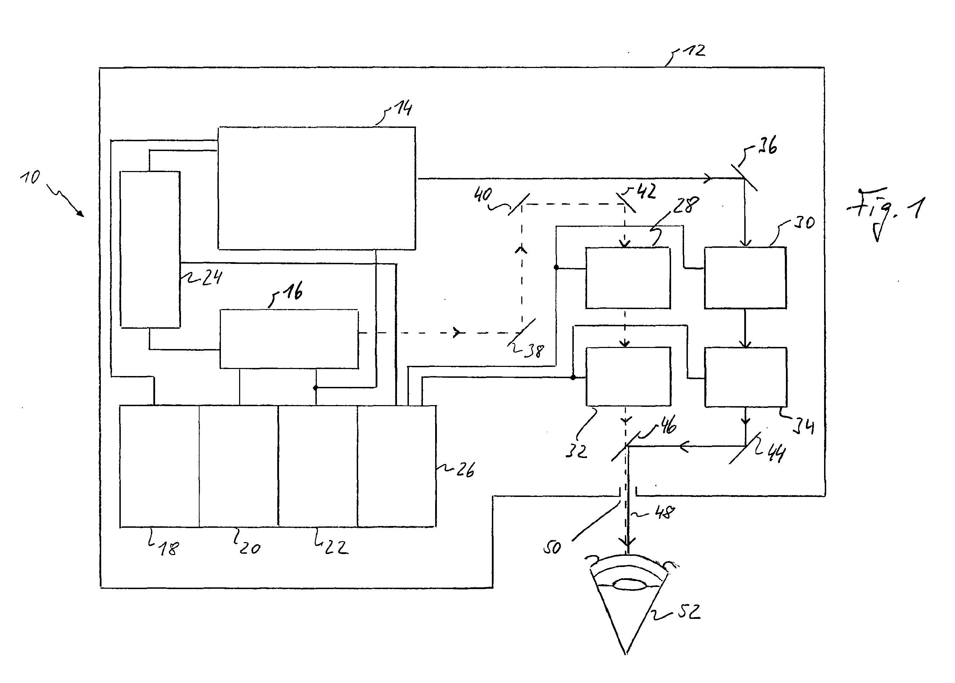

Die in Figur 1 gezeigte Lasereinrichtung - dort allgemein mit 10 bezeichnet - umfasst ein Gehäuse 12, in welchem zwei gesonderte Laserstrahlungsquellen untergebracht sind, nämlich eine für die Anbringung eines Hornhautschnitts vorgesehene Laserstrahlungsquelle 14 sowie eine für die anschließende Photoablation von Hornhautmaterial vorgesehene Laserstrahlungsquelle 16. Die Laserstrahlungsquelle 14 emittiert gepulste UV-Laserstrahlung, beispielsweise mit einer Wellenlänge von 193 nm. Sie kann von einem bei der gewünschten UV-Wellenlänge emittierenden Excimerlaser (vorzugsweise einem ArF-Excimerlaser) oder einem bei einer anderen Wellenlänge emittierenden Laser gebildet sein, dem eine nicht näher dargestellte Frequenzwandlerstufe zur Konvertierung der Strahlungswellenlänge auf die gewünschte UV-Wellenlänge nachgeschaltet ist. Hier ist es insbesondere vorstellbar, einen im infraroten Bereich strahlenden Femtosekunden-Faserlaser oder einen Nanosekunden-Festkörperlaser zu verwenden. Der Begriff Laserstrahlungsquelle, so wie er hier verwendet wird, umfasst die eigentliche Strahlungsquelle als solche und gegebenenfalls auch einen Frequenzwandler, sofern dieser benötigt wird, um die gewünschte Wellenlänge für den jeweiligen Operationsschritt zu erzeugen.

Die Laserstrahlungsquelle 16 ist dagegen ein im kurzwelligen infraroten Bereich strahlender Laser, vorzugsweise ein Femtosekunden-Faserlaser. Die abgestrahlte Wellenlänge der Laserstrahlungsquelle 16 liegt vorzugsweise im Bereich zwischen 1000 und 1080 nm.

Jeder der beiden Laserstrahlungsquellen 14, 16 ist eine eigene Betriebsenergieversorgungseinheit 18 bzw. 20 zugeordnet. Eine Kühleinheit 22 sorgt für die nötige Kühlung der Laserstrahlungsquellen 14, 16. Eine mit beiden Laserstrahlungsquellen 14, 16 gekoppelte Steuereinheit 24 steuert den Betrieb der Laserstrahlungsquellen 14, 16. Eine Recheneinheit 26 berechnet für die Durchführung der Laserbehandlung erforderliche Daten und speist mit diesen die Steuereinheit 24 sowie Ablenkeinheiten 28, 30 und Fokussiereinheiten 32, 34. Gemäß der Darstellung der Figur 1 sind die vorgenannten Komponenten alle in dem Gehäuse 12 zusammengefasst. Es versteht sich freilich, dass die Betriebsenergieversorgungseinheiten 18, 20 oder/und die Kühleinheit 22 oder/und die Steuereinheit 24 oder/und die Recheneinheit 26 in einem gesonderten Gehäuse untergebracht sein können. Jedoch sind die Ablenk- und Fokussiereinheiten 28, 30, 32, 34 sowie die übrigen für die Führung der Laserstrahlung benötigten optischen Komponenten zusammen mit den Strahlungsquellen in dem Gehäuse 12 untergebracht. Diese sonstigen optischen Komponenten umfassen im gezeigten Beispielfall mehrere Umlenkspiegel 36, 38, 40, 42, 44 sowie einen dichroitischen Spiegel 46. Letzterer bewirkt eine Zusammenführung der optischen Pfade der Laserstrahlung der beiden Laserstrahlungsquellen 14, 16. Bei dem Ausführungsbeispiel der Figur 1 ist der dichroitische Spiegel 46 im Strahlengang nach den beiden Fokussiereinheiten 32, 34 angeordnet. Vor dem dichroitischen Spiegel 46 verlaufen die optischen Pfade des Lichts der beiden Laserstrahlungsquellen 14, 16 getrennt, das heißt, es ist je eine Ablenkeinheit und je eine Fokussiereinheit für das Licht jeder Laserstrahlungsquelle 14, 16 vorgesehen. Die Zahl der Umlenkspiegel kann selbstverständlich abhängig von der Wegführung der Laserstrahlung variieren.

Nach der Zusammenführung der optischen Pfade verläuft das Licht der jeweils aktiven Laserstrahlungsquelle 14 oder 16 längs eines gemeinsamen optischen Pfadabschnitts 28 durch ein Austrittsfenster 50 der Gehäuseeinheit 12 und trifft auf die Hornhaut des zu operierenden Auges (in Figur 1 mit 52 bezeichnet). Durch geeignete Steuerung der Ablenkeinheiten 28, 30 und der Fokussiereinheiten 32, 34 wird der jeweilige Laserstrahl entlang einer gewünschten Schnittfläche beziehungsweise entlang einer gewünschten Ablationsfläche bewegt und dabei auf einen gewünschten Wirkpunkt fokussiert. Bei der Anbringung des Hornhautschnitts wird das Laserlicht auf einen Ort unterhalb der Hornhautoberfläche fokussiert; bei der Photoablation dagegen wird das Laserlicht auf die Oberfläche des nach Wegklappen des Flaps freigelegten Hornhautgewebes gerichtet. Geeignete Ablenk- und Fokussiereinheiten sind an sich bekannt; es muss daher hier nicht näher auf sie eingegangen werden.

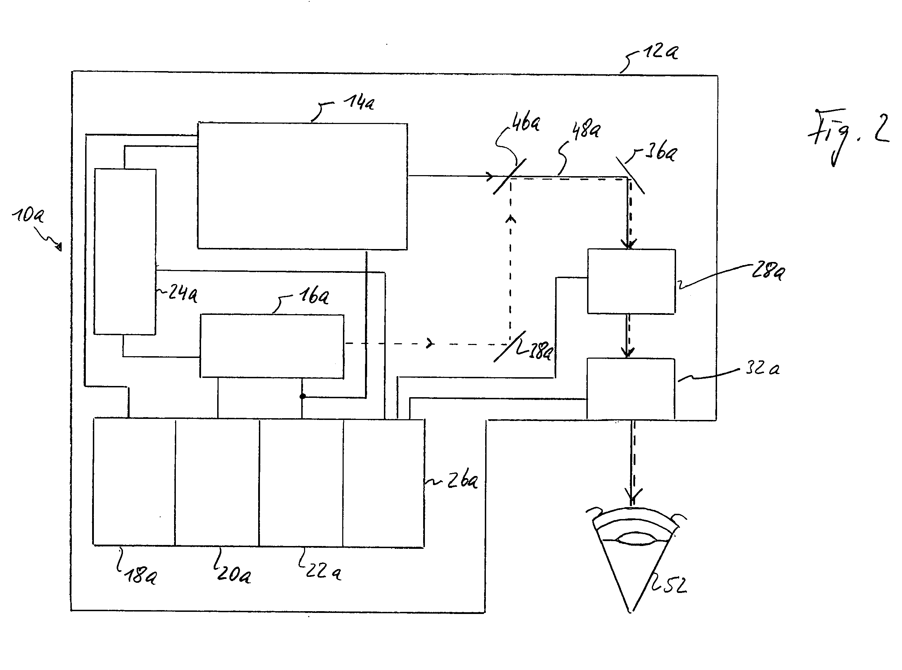

In Figur 2 sind gleiche oder gleichwirkende Komponenten mit gleichen Bezugszeichen bezeichnet wie in Figur 1, jedoch ergänzt durch einen Kleinbuchstaben. Zur Vermeidung von Wiederholungen sollen hier lediglich die Unterschiede zum Ausführungsbeispiel der Figur 1 herausgearbeitet werden. Diese Unterschiede bestehen im Wesentlichen darin, dass bei dem Ausführungsbeispiel der Figur 2 die optischen Wege des Lichts der beiden Laserstrahlungsquellen 14a, 16a bereits vor den Ablenk- und Fokussiermitteln zusammengeführt werden. Dementsprechend kann eine gemeinsame Ablenkeinheit und eine gemeinsame Fokussiereinheit verwendet werden; diese sind in Figur 2 mit 28a und 32a bezeichnet. Da die Laserstrahlung der beiden Laserstrahlungsquellen 14a, 16a zu jeweils unterschiedlichen Zeitpunkten während der Operation benötigt wird, ist es möglich, mit einer einzigen Ablenkeinheit und einer einzigen Fokussiereinheit auszukommen, sofern diese in Anpassung an die unterschiedliche Strahlung und die unterschiedliche Funktion der Strahlung geeignet steuerbar sind.

Die beiden in den Figuren 1 und 2 gezeigten Lasersysteme bieten eine Integration zweier Laserstrahlquellen in ein einziges Gerätesystem, das gemeinsame Komponenten oder Module verwenden kann und dementsprechend vergleichsweise kostengünstig herstellbar ist. Aufgrund dieser Integration beanspruchen die in den Figuren 1 und 2 gezeigten Lasersysteme weniger Platz als bisherige Lösungen, bei denen getrennte Lasersysteme für die Anbringung des Hornhautschnitts und für die Photoablation des Hornhautgewebes benutzt werden. Der Operationsvorgang kann insgesamt beschleunigt werden, weil eine Umlagerung des Patienten zwischen solchen getrennten Lasersystemen nicht mehr erforderlich ist. Hierdurch wird die Operation nicht nur für den Patienten sondern auch für den Operateur bequemer.

## Patentansprüche

1. Lasereinrichtung (10) für die refraktive Augenbehandlung, umfassend
- eine erste Laserstrahlungsquelle (14), deren bereitgestellte Laserstrahlung auf die Anbringung eines Hornhautschnitts abgestimmt ist,
- eine zweite Laserstrahlungsquelle (16), deren bereitgestellte Laserstrahlung auf die Ablation von Hornhautmaterial abgestimmt ist, und
- Optikmittel (28-46) zur Führung und Formung der Laserstrahlung beider Laserstrahlungsquellen,
**dadurch gekennzeichnet, dass** die Optikmittel einen der Laserstrahlung beider Laserstrahlungsquellen gemeinsam zugeordneten optischen Pfadabschnitt (48) definieren, über welchen die Laserstrahlung jeder Laserstrahlungsquelle auf ein zu behandelndes Auge (52) trifft.

2. Lasereinrichtung nach Anspruch 1 oder dessen Oberbegriff,
**dadurch gekennzeichnet, dass** die beiden Laserstrahlungsquellen (14, 16) in gemeinsamen Gehäusemitteln (12) untergebracht sind.

3. Lasereinrichtung nach Anspruch 1 oder 2,
**dadurch gekennzeichnet, dass** die Optikmittel (28-46) in Zuordnung zu jeder der Laserstrahlungsquellen (14, 16) je eine Strahlfokussiereinheit (32, 34) umfassen und der gemeinsame optische Pfadabschnitt (48) im Strahlengang nach den beiden Strahlfokussiereinheiten beginnt.
